# EUROPEAN PATENT SPECIFICATION

(11) **EP 4 190 239 B1**
(45) Date of publication and mention of the grant of the patent: **19.03.2025**
(21) Application number: 21211744.4
(22) Date of filing: 01.12.2021
(51) Int. Cl.: A61B 5/287, A61B 5/361, A61B 5/00, A61B 5/35, A61B 5/367

(54) **METHOD FOR ANALYSING AN INTRACARDIAC ELECTROGRAM**
VERFAHREN ZUR ANALYSE EINES INTRAKARDIALEN ELEKTROGRAMMS
PROCÉDÉ D'ANALYSE D'ÉLECTROGRAMME INTRACARDIAQUE

(43) Date of publication of application: 07.06.2023
(73) Proprietor: CathVision ApS, 2200 Copenhagen (DK)
(72) Inventor: PAAMAND, Rune, 3050 Humlebaek (DK); VALVIK, Martin, 4700 Naestved (DK)
(74) Representative: Gottschald Patentanwälte Partnerschaft mbB

(56) References cited:
- WO-A1-02/056783
- US-A1- 2013 345 577
- US-A1- 2014 343 388
- US-A1- 2017 181 655
- US-A1- 2019 015 007
- US-A1- 2020 138 334
- US-A1- 2021 177 293
- US-A1- 2021 228 139

## Description

The present invention relates to a method for analysing an intracardiac electrogram according to the general part of claim 1 and to a control system according to claim 14.

The present method is particularly concerned with atrial fibrillation and atrial flutter. Electrically, atrial fibrillation is chaotic activation of muscle cells of the atria. During atrial fibrillation, the atria only minimally contribute to the function of the heart. Atrial fibrillation, therefore, reduces the output of the heart but is not imminently dangerous. However, when becoming chronical, atrial fibrillation is correlated with increased morbidity and mortality. One treatment option for atrial fibrillation is ablation therapy. Ablation is the destruction of the cells that allow electrical wave re-entry to reduce chaotic activation of the atrial muscle cells.

A recommended treatment for atrial fibrillation includes pulmonary vein isolation. Pulmonary vein isolation can be performed with various ablation techniques, including radio frequency ablation, cryoballoon ablation and pulsed-field ablation. While these techniques apply energy differently, their common endpoint is the isolation of the electrical activity of the pulmonary veins from the rest of the atrium.

Generally, ablation therapy is successful, if the targeted location is electrically isolated from the rest of the heart. There are different methods for assessing the success of the ablation therapy. These include costly 3D mapping of ablation points, measurements of force or temperature during ablation and electrogram analysis.

In a known method (EP 3 139 828 A1) pulmonary vein isolation is determined based on the morphology of local activations measured in the pulmonary veins. This method yields good results, however, it can still be improved. Further, the method detects local activations based on their timing relative to the activations of the atria. During atrial fibrillation, this time dependency between local activations in the pulmonary veins and the atrial beats can be broken, making it hard to differentiate between local activations and far field potentials or artefacts. From US 2014/343388 A1 a basket mapping catheter used in an analysis using the order of electrodes is known. It discloses a method and system for mapping an anatomical structure includes sensing activation signals of intrinsic physiological activity with a plurality of mapping electrodes disposed in or near the anatomical structure, each of the plurality of mapping electrodes having an electrode location. A vector field map which represents a direction of propagation of the activation signals at each electrode location is generated to identify a signature pattern and a location in the vector field map according to at least one vector field template. A target location of the identified signature pattern is identified according to a corresponding electrode location.

It is a challenge to improve on the mentioned prior art. In particular, the electrical status of a target region in the heart, preferably in the pulmonary veins, should be detected more reliably.

The invention is based on the problem of improving the known method such that a further optimization regarding the named challenge is reached.

The above-noted object is solved by the features of the characterizing part of claim 1.

The main realization of the present invention is that with catheters that do not have a localization system, it is still possible to derive spatial information and use this spatial information to improve the determination of an electrical status of a target region in the heart or the pulmonary veins. While the use of spatial information is known from mapping systems, sometimes also called localization systems, which use an external reference to localize the electrodes in a fixed coordinate system, the proposed method uses relative spatial information. This relative spatial information includes the order of the electrodes on the catheter. With this order it is not possible to derive an absolute position of the electrodes, but it can be used to find the propagation of wavefronts in the channels of an intracardiac electrogram. It is therefore possible to use spatial information in a system without an external spatial reference.

In detail, it is proposed that the order of the electrodes on the catheter is provided to the control system or saved in a memory of the control system and that in the analysis routine the control system determines the electrical status of the target region based on spatial information including the order of placement of the electrodes on the catheter.

One embodiment describes a number of preferred features that further define the use of the preferred method. These features primarily describe how the idea of using the order of the electrodes as spatial information can be used in various systems and use cases where no other localization is provided.

One embodiment relates to the difference between the measurement of channels and the provision of the spatial information.

Preferred catheters used to measure the intracardiac electrogram are object of one embodiment. Particularly, ablation catheters may be used to measure the electrogram prior to ablation and/or after ablation to assess the necessity of ablation and/or its success. Using the same catheter for the measurement and ablation leads to an effective, fast and cost-efficient approach for ablation. Following this train of thought, one embodiment describes preferred applications for the proposed method in the pulmonary veins. The entrances of the pulmonary veins are main targets for ablation therapy. Enabling an efficient measurement of the electrical isolation status of the pulmonary vein decreases surgery time and increases the therapy success for the patient.

The success of ablation therapy in the pulmonary veins can be determined by analysing local activations in the pulmonary veins. If the activations spread towards the left atrium and activate the left atrium, the pulmonary veins are not fully isolated. If the activations are disconnected from the activation of the atrium, the isolation is likely successful. Conclusions about the isolation can be drawn from the waveform of the local activations. For that, it is necessary to differentiate between actual local activations and artefacts or far field interference, for example activations of the left atrium that spread towards the pulmonary veins. One difference between far field interference or artefacts and local activations is that local activations spread around the pulmonary veins, while far field interference and artefacts mostly do not.

One embodiment relates to classifying activation candidates. If only or mainly local activations are grouped together, further analysis can be performed on that group of activations.

The claimed invention is based on using timing sequence(s) and/or timing difference(s) between activations detected in different channels together with the spatial information to determine propagation of waves in the target region and thereby differentiate between local activations and far field interference.

By combining the spatial information with features derived from the channels independently, an improved determination of the electrical status becomes possible.

A measurement of the time between an activation and causing or caused by said activation like the onset of the heartbeat or the like, can vary between channels and conveys information about the electrical activity at the target region. This information can be used to judge the electrical status of the target region.

As has been mentioned, the wave form of local activations is indicative of the isolation of a target region. Further the change of the waveform can be used to identify components of the wave that are subject to fast changes during propagation and components that are stable. In this way, further information about the waveform can be gained.

A further advantage of using the order of electrodes on the catheter as spatial information is that it becomes possible to estimate the quality of the signal of a channel by comparing it to its neighbours and determining which signal components are local, physiological noise and which components are real noise.

Embodiments relate to a specific algorithm for analysing the waveform of local activations. With the proposed improved method able to better detect local activations, the electrical isolation status of in particular the pulmonary veins can be estimated with more precision.

Another teaching according to claim 14, which is of equal importance, relates to a control system configured to perform the method according to the first teaching, wherein the control system is configured to receive and/or measure the intracardiac electrogram, preferably wherein the control system is connectable to the catheter.

All explanations given with respect to the proposed method are fully applicable to the control system.

In the following, an embodiment of the invention is explained with respect to the drawing. The drawing shows in
- Fig. 1: schematically a human heart and the use of the proposed control system during surgery to measure an intracardiac electrogram und
- Fig. 2: a coronary sinus electrogram, an intracardiac electrogram and schematically the analysis and classification routines

The proposed method is used for analysing an intracardiac electrogram 1. It can particularly well be used for detecting a pulmonary vein 2 isolation status. The intracardiac electrogram 1 is analysed via a control system 3. The intracardiac electrogram 1 has been recorded at a, in particular predetermined, target region 4 via a catheter 5 inserted into a human body.

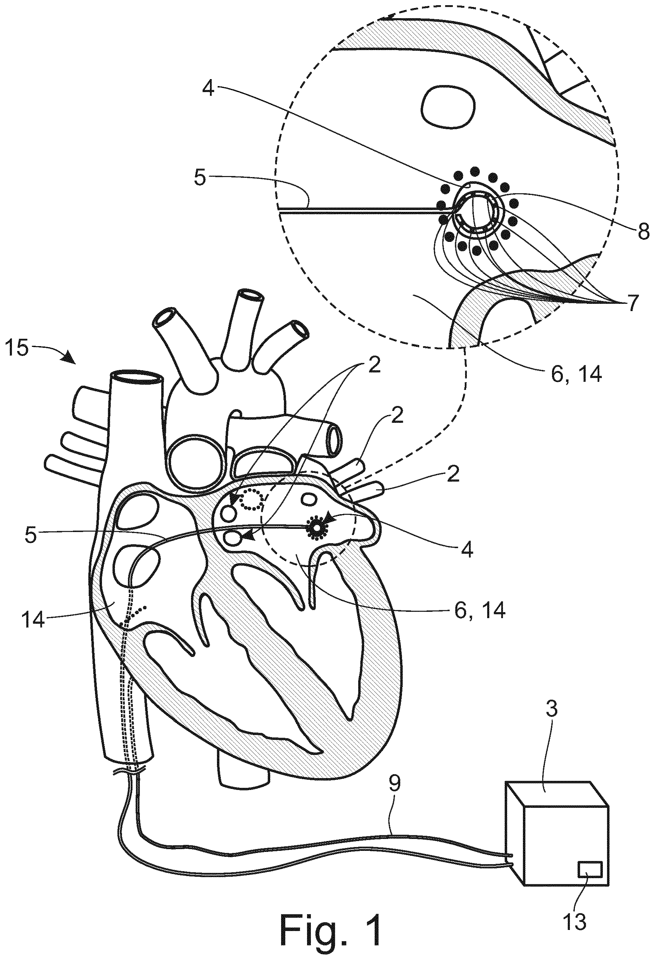

Fig. 1 shows in a very schematic manner how the control system 3 can interact with a catheter 5 inserted for example through the femoral vein into the left atrium 6 and from there into a pulmonary vein 2. The catheter 5 may be a cryoballoon ablation catheter 5 that inflates at the entrance of a pulmonary vein 2 and is used to isolate the pulmonary vein 2.

The catheter 5 comprises multiple electrodes 7. In the shown embodiment, the catheter 5 comprises a spiral strand 8 with electrodes 7 that can be put into the pulmonary vein 2 to measure the intracardiac electrogram 1 before, during and after ablation.

The control system 3 may further interact with a coronary sinus electrode 9, as shown in fig. 1. Fig. 1 shows only the sleeve of the coronary sinus electrode 9 entering the heart, the remainder of the coronary sinus electrode 9 is located in a plane outside the visible plane.

In the proposed method, multiple channels 10 of the intracardiac electrogram 1 have been recorded by the electrodes 7. If the intracardiac electrogram 1 is a bipolar electrogram, it may have been measured between the electrodes 7. The electrodes 7 have a fixed order of placement on the catheter 5. It may be noted that all method steps described as features of the intracardiac electrogram 1 and not as part of the proposed method may alternatively be part of the proposed method.

In an analysis routine 11, the control system 3 determines an electrical status of the target region 4 by analysing at least three of the channels 10. This analysis can also be done, preferably in real time, during a surgery and/or by analysing an ongoing measurement of an intracardiac electrogram 1.

The preferred use case of the present method is to analyse the electrical isolation status of the target region 4 with regards to, preferably during surgery for, atrial fibrillation. As will be further explained, it is one advantage of the proposed method that the analysis of the electrical isolation status of in particular the pulmonary veins 2 may be done during an episode of atrial fibrillation.

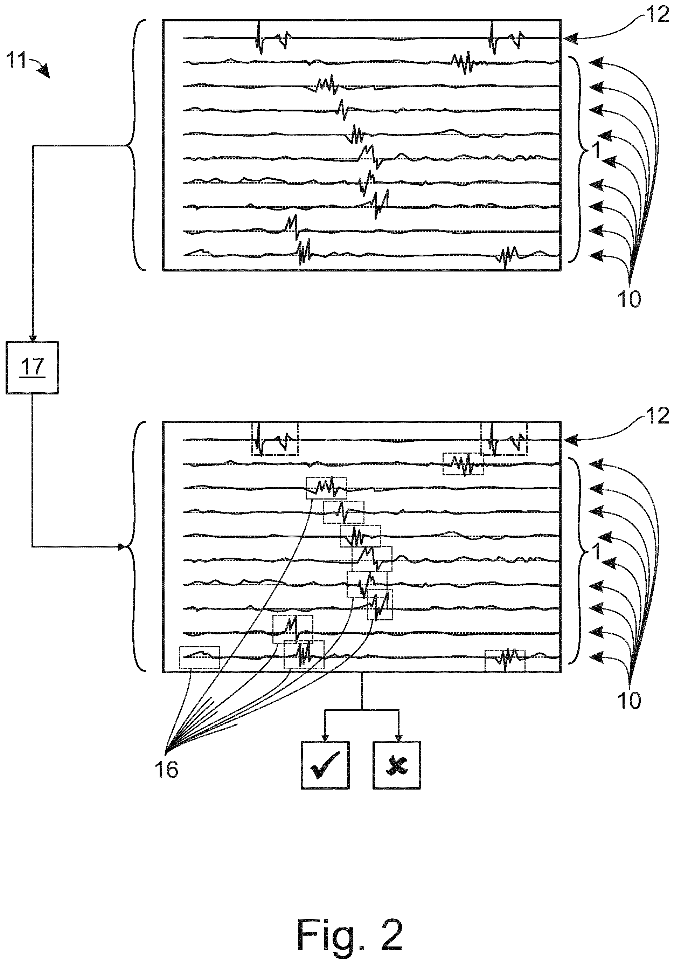

Fig. 2 shows the channels 10 of the intracardiac electrogram 1 and a coronary sinus electrogram 12. There may also be more than one coronary sinus electrode 9 and more than one coronary sinus electrogram 12. It may be the case that one of the coronary sinus electrograms 12 is chosen for the present method or that more than one is used.

The control system 3 may be a local unit with a processor, possibly a user interface and the like, as shown in fig. 1. It may also comprise in one embodiment a cloud processor. It is therefore not necessary for the control system 3 to be confined to a single device. The control system 3 can have a memory 13 and other necessary components, in particular it may comprise an interface for the catheter 5 and/or the coronary sinus electrode 9.

The term "predetermined target region" means that the target region 4 has been selected by will and is not random. The predetermined target region 4 can be a pulmonary vein 2 which is to be isolated or which has been isolated and should now be measured to confirm the necessity of isolation or the success of isolation. In that case the exact location of the catheter 5 inside the pulmonary vein 2 is not critical as the whole vein should be electrically isolated from the left atrium 6. The predetermined target region 4 may also be a region associated with a rotor driving atrial fibrillation, which has been identified by a mapping system unrelated to the proposed analysis routine 11. Systems that electrically map the whole atrium 14 or both atria 14 usually do not measure predetermined regions, and instead measure a multitude of regions throughout the atria 14 successively.

The term "channel" relates to a time row of electrical values, preferably voltages, measured by a unipolar electrode 7 or between at least two electrodes 7. Here and preferably, the channels 10 have been measured between neighbouring electrodes 7.

The term "intracardiac" is to be understood broadly and relates to measurements inside the human heart 15 and in direct vicinity to it, for example in the pulmonary veins 2.

A routine, like the analysis routine 11, is a sum of steps with a purpose. The routine and the steps may be fully implemented in the control system 3 as software but may also comprise physical measurements and the like. Everything described as part of the routine serves the purpose of the routine, such that other calculations that may be done at the same time as the routine, but that are unrelated to the purpose of the routine, are not part of the routine. The steps of a routine can be done at separate times with space in-between, concurrently and in any sensible order.

It is essential here that the order of the electrodes 7 on the catheter 5 is provided to the control system 3 or saved in a memory 13 of the control system 3 and that in the analysis routine 11 the control system 3 determines the electrical status of the target region 4 based on relative spatial information including the order of placement of the electrodes 7 on the catheter 5.

Here and preferably, the order of the electrodes 7 implicitly or explicitly gives information about the order of the channels 10. It is generally known from so-called mapping systems or localization systems to use absolute spatial information from a localization system with an external reference. This can be a magnetic localization system, possible aided by bioimpedance measurements between a patch on the chest of the patient and the catheter 5. The present method does not rely on such an external reference. The main realization of the present invention is that it is possible to still use some of the advantages of a localization system in form of the relative spatial information by using the order of the electrodes 7 as, albeit relative, spatial information. Here and preferably, the relative spatial information is solely based on the order of the electrodes 7. This order is fixed information based on the manufacturing of the catheter 5 and may be derived from the standard numbering of the channels 10 provided to the control system 3 simply by the order of the channel 10 measurements, for example. The control system 3 of the proposed method does preferably not use any absolute spatial information.

The electrical status is an electrical isolation status of the target region 4 relative to the left and/or right atrium 14 and/or the rest of the left and/or right atrium 14. It may also comprise characteristics of the electrical conduction of the target region 4 itself.

Here and in the following the term "determines based on" means that a feature, characteristic, property or the like is determined by using, in a not completely irrelevant manner, the information that the determination is "based on". In every case it is preferably so that the determination is based mainly on the respective information.

Here and preferably, the intracardiac electrogram 1 has been recorded during a surgical procedure without electrical mapping of an atrium 14 by a localization system during the recording of the intracardiac electrogram 1 based on the recorded electrogram. A mapping system may be present additionally and independently, but in the preferred embodiment it is not. Preferably, that is the case during the whole surgical procedure.

In the analysis routine 11 the control system 3 preferably determines the electrical status by analysing concurrently measured channels 10 of the electrogram at the target region 4 without using electrogram channels 10 measured after repositioning of the catheter 5. It is one advantage of the present method that complex repositioning of the catheter 5 and measuring of a multitude of regions is not necessary. It may be mentioned that the target region 4 is not the whole left atrium 6 but significantly smaller.

Here and preferably, the spatial information is represented in a coordinate system without a reference point external to the body, in particular, without a reference point determined from a magnetic and/or bioimpedance measurement between an electrode 7 or magnetic localization apparatus outside the human body and the catheter 5 or even without a coordinate system.

Imaging system like a CT-Scanner and the like are not included in the above if they do not provide a fixed reference system that the catheter 5 is related to.

Here and preferably the channels 10 have been measured concurrently over at least partially, preferably completely, the same time period. The spatial information may be mainly or only passive spatial information that has not been measured over time, preferably not measured at all. In this last case, the term "measured" may not be understood broadly. Determining the order of the electrodes 7 from the numbering of the channels 10 or providing it from an external source where the order has been manually entered does not count as measuring the order. Preferably, the spatial information has not been measured in a reference coordinate system and/or does not contain a reference coordinate system nor is related to one.

The spatial information may be constant during the measurement of the intracardiac electrogram. Here, the order of the electrodes 7 is constant, therefore, it may be the case that the spatial information does not or does not relevantly change during the proceeding and/or it may be the case that the spatial information does not comprise information that is updated regularly, in particular in real time or near real time.

As already mentioned and partially shown in fig. 1 it may be the case that the catheter 5 is an ablation catheter 5, in particular a cryoballoon catheter 5, and/or a spiral catheter 5, preferably, that the catheter 5 comprises only a single strand 8 on which the electrodes 7 are located. An example of a preferred catheter 5 is the LASSO^{™} catheter 5. Alternatively, the catheter may be a multistrand catheter 5. In that case, the order of placement of the electrodes 7 may relate to only one strand 8 or to the multiple strands 8.

Here and preferably, it is the case that the electrical status is an electrical isolation status. Preferably, the target region 4 is located inside a pulmonary vein 2 and the electrical isolation status is an electrical isolation status of the pulmonary vein 2. Pulmonary vein isolation is one of the main ablation therapies used. The intracardiac electrogram 1 may have been recorded after an isolation of a pulmonary vein 2 inside the isolated pulmonary vein 2 such that in the analysis routine 11 the control system 3 determines the pulmonary vein 2 isolation status. As stated above, the intracardiac electrogram 1 may also be recorded while running the analysis routine 11. When speaking about an isolation of the pulmonary veins 2, it is understood that an attempt on isolation is meant and the success is open to determination.

The electrical isolation status may be a binary yes/no decision as shown in fig. 2 or a probability of isolation of the target region 4 or any other sensible measure of success.

It is the case that in the analysis routine 11 the control system 3 identifies activation candidates 16 in at least three of the channels 10, in particular in all channels 10. An activation candidate 16 is a section of a channel 10 that is identified by an algorithm as potentially being a local activation. The candidates may be identified using a peak detection algorithm, a waveform detection like a correlation or the like.

In the analysis routine 11 the control system 3 performs a classification routine 17 for at least some, preferably all, of the activation candidates 16 classifying the activation candidates 16 into groups. The groups comprise at least one group assigned to local activations and/or at least one group assigned to far field interference, in particular atrial activations, and/or at least one group assigned to noise. The control system 3 can therefore separate the activation candidates 16 into real activations that do not have their origin in or near the target region 4, namely such activations that are far field interference, possibly artefacts, for example from pacing and noise, in particular false positives, namely signals that did not originate from a physiological activation and local activations. The local activations may be the main target of the proposed method and can be used to judge the electrical isolation status.

As will be further explained, channels 10 with low quality may be ignored in the classification routine 17 sometimes.

When saying that the group is assigned to a certain type of signal it is meant that this type of signal or a subset of this type of signal is the main focus of said group and that the classification aims at, in particular solely, grouping occurrences of said type of signal into said group.

By placing a, particularly spiral, catheter 5 inside a pulmonary vein 2, the propagation of the local activation around the pulmonary vein 2 can be detected. This is based on the realization that, in particular in the pulmonary veins 2, far field interference like atrial beats and artefacts like pacing artefacts will reach most of the pulmonary vein 2 at the same time, particularly if the electrodes 7 are arranged more or less orthogonal to the extension of the pulmonary vein 2 at the circumference of the pulmonary vein 2 for example by using a circular or spiral catheter 5 or strand 8. Local activations however propagate around the pulmonary veins 2. This propagation can be measured as a time difference between the onset or peaks of the local activations. Fig. 2 shows local activations in some channels 10 with their respective different timings.

Here and preferably, the identification of the local activations during the classification routine 17 is at least partly based on detecting this timing difference.

In detail, it may be the case that in the classification routine 17 the control system 3 identifies a timing sequence and/or a timing difference between at least two, preferably at least three, more preferably at least four, activation candidates 16 of different channels 10 based on the spatial information, in particular the order of the electrodes 7, preferably to differentiate between local activations and far field interference.

Further, it is possible that in the classification routine 17 the control system 3 differentiates between local activations and far field interference by identifying a propagation sequence along the order of the electrodes 7 and classifying activation candidates 16 as local activations if the control system 3 identifies a propagation sequence and/or as far field interference if the control system 3 does not identify a propagation sequence.

It is interesting to note that during atrial fibrillation the time relation between atrial beats and local activations in the pulmonary veins 2 may be broken. Know algorithms therefore have trouble differentiating local activations from far field interference, particularly atrial activations. The above can be used to aid this differentiation. Therefore, the proposed method works well during episodes of atrial fibrillation. By using this method, a surgeon may not have to cardiovert a patients atria 14 to normal rhythm to measure the success of the isolation procedure.

In the preferred embodiment, in the analysis routine 11, the control system 3 determines the electrical status based on a set of features determined from an analysis of channels 10 independently and a set of features determined from the analysis based on the spatial information, in particular the order of the electrodes 7. A possible set of features determined independently for each channel 10 is further described below.

In the analysis routine 11, in particular in the classification routine 17, the control system 3 may determine the electrical status based on a local activation time difference between, in particular neighbouring, channels 10 based on the order of the electrodes 7. The activation can be an atrial activation detected in the coronary sinus electrogram 12 and the local activation time may be measured as a time difference between a certain event, for example the onset of an activation candidate 16, and the atrial activation. Preferably, the local activation time is measured relative to any coronary activation, in particular measured by a coronary sinus electrode 9- The coronary activation may be an atrial activation.

It may be reiterated that neighbouring channels 10 are preferably those that share an electrode 7 in usual bipolar measurements. Additionally or alternatively, neighbouring channels 10 may be spaced apart, for example by one electrode 7. In particular, some catheters 5 may have electrode 7 pairs with a space between the electrodes 7 of a pair being less than the space between electrodes 7 of neighbouring pairs.

Further, it is possible that in the analysis routine 11, in particular in the classification routine 17, the control system 3 determines the electrical status and/or the classification of an activation candidate 16 based on a change of an activation or activation candidate 16 over its spatial propagation whereby the spatial propagation is determined based on the spatial information, in particular the order of the electrodes 7, and the timing of the activation or activation candidate 16. This change can be a change of the waveform.

Another advantage of using the relative spatial information is the possibility of estimating the quality of a channel 10. Here and preferably in a quality estimation routine, preferably prior to the analysis routine 11, the control system 3 estimates a quality indicator, in particular a signal-to-noise ratio, of a channel 10, preferably of all channels 10, based on the spatial information, in particular the order of the electrodes 7. This quality indicator may be used to remove some channels 10 from further analysis, preferably in the analysis routine 11, in particular in the classification routine 17. Preferably, in the quality estimation routine, the control system 3 estimates the quality indicator based on a comparison of neighbouring channels 10, in particular waveforms of activations or activation candidates 16 of neighbouring channels 10. Additionally or alternatively, the control system 3, in the analysis routine 11, uses only a subset of the channels 10 selected based on the quality indicator to determine the electrical status of the target region 4.

The subset here and preferably contains at least two, preferably at least 3, channels 10, but less than the maximum of the channels 10.

As already mentioned, to determine the electrical isolation status, in the analysis routine 11, the control system 3 may analyse a morphology of the local activations to determine the electrical isolation status of the target region 4. Preferably it is the case that in the analysis routine 11 the control system 3 classifies the local activations into morphology groups and preferably determines based on the distribution of local activations across the groups the electrical isolation status.

Here and preferably, in the analysis routine 11, the control system 3 classifies the local activations into the morphology groups based on a number of characteristic peaks of the local activation. For this, not every plateau of a local activation necessarily counts as a characteristic peak. Preferably, the control system 3 classifies a peak with at least a predetermined amplitude and/or with at least a predetermined slope and/or with at most a predetermined slope and/or with at least a predetermined minimum peak distance and/or with at most a predetermined maximum peak distance and/or based on a peak morphology, in particular a minimum and/or maximum peak angle, as a characteristic peak.

Further, the morphology groups may comprise a group for local activations with a single characteristic peak and/or exactly two characteristic peaks and/or exactly three characteristic peaks and/or more than three characteristic peaks and/or at least two characteristic peaks separated by a predetermined time.

The channels 10 may be highlighted, in particular coloured, based on the results or details of the analysis routine 11 by the control system 3 on a display of the control system 3. This is to draw attention of the physician to the impact and relation of the channels 10 on the model output and aid in interpretability.

According to a further teaching with equal importance, a control system 3 configured to perform the method according to the proposed method, wherein the control system 3 is configured to receive and/or measure the intracardiac electrogram 1, preferably wherein the control system 3 is connectable to the catheter 5, is proposed

All explanations given with regard to the proposed method are fully applicable to the control system 3.

### Reference numerals

- 1: intracardiac electrogram
- 2: pulmonary vein
- 3: control system
- 4: target region
- 5: catheter
- 6: left atrium
- 7: electrode
- 8: strand
- 9: coronary sinus electrode
- 10: channel
- 11: analysis routine
- 12: coronary sinus electrogram
- 13: memory
- 14: atrium
- 15: human heart
- 16: activation candidate
- 17: classification routine

## Claims

1. Method for analysing an intracardiac electrogram (1), in particular for detecting a pulmonary vein (2) isolation status, via a control system (3), wherein the intracardiac electrogram (1) has been recorded at a, in particular predetermined, target region (4) via a catheter (5) inserted into a human body, wherein the catheter (5) comprises multiple electrodes (7), wherein multiple channels (10) of the intracardiac electrogram (1) have been recorded by, preferably between, the electrodes (7), wherein the electrodes (7) have a fixed order of placement on the catheter (5),
wherein in an analysis routine (11), the control system (3) determines an electrical status of the target region (4) by analysing at least three of the channels (10),
wherein the order of the electrodes (7) on the catheter (5) is provided to the control system (3) or saved in a memory (13) of the control system (3) and that in the analysis routine (11) the control system (3) determines the electrical status of the target region (4) based on relative spatial information including the order of placement of the electrodes (7) on the catheter (5),
wherein in the analysis routine (11) the control system (3) identifies activation candidates (16) in at least three of the channels (10), wherein in the analysis routine (11) the control system (3) performs a classification routine (17) for at least some of the activation candidates (16) classifying the activation candidates (16) into groups, the groups comprising at least one group assigned to local activations and/or at least one group assigned to far field interference, **characterized in**
**that** in the classification routine (17) the control system (3) identifies a timing sequence and/or a timing difference between at least two activation candidates (16) of different channels (10) based on the spatial information to differentiate between local activations and far field interference and
**that** the electrical status is an electrical isolation status of the target region (4) relative to the left and/or right atrium (14) and/or the rest of the left and/or right atrium (14).

2. Method according to claim 1, **characterized in that** the channels (10) have been measured concurrently over at least partially the same time period.

3. Method according to claim 1 or 2, **characterized in that** the catheter (5) comprises only a single strand (8) on which the electrodes (7) are located.

4. Method according one of the preceding claims, **characterized in that** in the analysis routine (11) the control system (3) performs the classification routine (17) for all of the activation candidates (16)

5. Method according to one of the preceding claims, **characterized in that** in the classification routine (17) the control system (3) identifies a timing sequence between at least three activation candidates (16) of different channels (10) based on the spatial information, and, that in the classification routine (17) the control system (3) differentiates between local activations and far field interference by identifying a propagation sequence along the order of the electrodes (7) and classifying activation candidates (16) as local activations if the control system (3) identifies a propagation sequence and as far field interference if the control system (3) does not identify a propagation sequence.

6. Method according to one of the preceding claims, **characterized in that** in the classification routine (17) the control system (3) determines the electrical status based on a local activation time difference between channels (10) based on the order of the electrodes (7).

7. Method according to claim 5 or 6, **characterized in that** in the analysis routine (11) the control system (3) determines the electrical status based on a change of an activation or activation candidate (16) over its spatial propagation whereby the spatial propagation is determined based on the spatial information and the timing of the activation or activation candidate (16).

8. Method according to one of the preceding claims, **characterized in that** in a quality estimation routine the control system (3) estimates a quality indicator of a channel (10) based on the spatial information.

9. Method according to claim 8, **characterized in that** in the quality estimation routine the control system (3) estimates the quality indicator based on a comparison of neighbouring channels (10), and, that the control system (3) in the analysis routine (11) uses only a subset of the channels (10) selected based on the quality indicator to determine the electrical status of the target region (4).

10. Method according to one of the preceding claims, **characterized in that** in the analysis routine (11) the control system (3) analyses a morphology of the local activations to determine the electrical isolation status of the target region (4).

11. Method according to claim 10, **characterized in that** in the analysis routine (11) the control system (3) classifies the local activations into morphology groups and determines based on the distribution of local activations across the groups the electrical isolation status.

12. Method according to claim 11, **characterized in that** in the analysis routine (11) the control system (3) classifies the local activations into the morphology groups based on a number of characteristic peaks of the local activation.

13. Method according to claim 11 or 12, **characterized in that** the morphology groups comprise a group for local activations with a single characteristic peak and exactly two characteristic peaks and at least two characteristic peaks separated by a predetermined time.

14. Control system, wherein the control system (3) is configured to receive and/or measure an intracardiac electrogram (1), wherein the control system (3) is a local unit with a processor and a user interface, wherein the control system (3) has a memory (13) and comprises an interface for a catheter (5) comprising multiple electrodes (7) with a fixed order of placement of the electrodes on the catheter (5), wherein the control system (3) is configured to perform the method according to one of the preceding claims, wherein the routines of the method are fully implemented in the control system (3) as software,
wherein the order of the electrodes (7) on the catheter (5) is saved in the memory (13) of the control system (3).

## Patentansprüche

1. Verfahren zum Analysieren eines intrakardialen Elektrogramms (1), insbesondere zum Detektieren des Isolationszustands einer Lungenvene (2), über ein Steuerungssystem (3), wobei das intrakardiale Elektrogramm (1) in einer insbesondere vorbestimmten Zielregion (4) über einen in einen menschlichen Körper eingeführten Katheter (5) aufgezeichnet worden ist, wobei der Katheter (5) mehrere Elektroden umfasst (7), wobei mehrere Kanäle (10) des intrakardialen Elektrogramms (1) von, vorzugsweise zwischen, den Elektroden (7) aufgezeichnet worden sind, wobei die Elektroden (7) eine feste Platzierungsreihenfolge an dem Katheter (5) aufweisen,
wobei in einer Analyseroutine (11) das Steuerungssystem (3) einen elektrischen Zustand der Zielregion (4) durch Analysieren von wenigstens drei der Kanäle (10) bestimmt, wobei die Reihenfolge der Elektroden (7) an dem Katheter (5) für das Steuerungssystem (3) bereitgestellt oder in einem Speicher (13) des Steuerungssystems (3) gespeichert wird und wobei in der Analyseroutine (11) das Steuerungssystem (3) den elektrischen Zustand der Zielregion (4) basierend auf relativen räumlichen Informationen bestimmt, was die Platzierungsreihenfolge der Elektroden (7) an dem Katheter (5) einschließt, wobei in der Analyseroutine (11) das Steuerungssystem (3) Aktivierungskandidaten (16) in wenigstens drei der Kanäle (10) identifiziert, wobei in der Analyseroutine (11) das Steuerungssystem (3) eine Klassifizierungsroutine (17) für wenigstens einige der Aktivierungskandidaten (16) durchführt, wobei die Aktivierungskandidaten (16) in Gruppen klassifiziert werden, wobei die Gruppen wenigstens eine Gruppe umfassen, die lokalen Aktivierungen zugewiesen ist und/oder wenigstens eine Gruppe, die Fernfeldinterferenzen zugewiesen ist, **dadurch gekennzeichnet,**
**dass** in der Klassifizierungsroutine (17) das Steuerungssystem (3) eine Timingsequenz und/oder eine Timingdifferenz zwischen wenigstens zwei Aktivierungskandidaten (16) unterschiedlicher Kanäle (10) basierend auf den räumlichen Informationen identifiziert, um zwischen lokalen Aktivierungen und Fernfeldinterferenzen zu unterscheiden und
**dass** der elektrische Zustand ein elektrischer Isolationszustand der Zielregion (4) relativ zum linken und/oder rechten Vorhof (14) und/oder dem restlichen Teil des linken und/oder rechten Vorhofs (14) ist.

2. Verfahren gemäß Anspruch 1, **dadurch gekennzeichnet, dass** die Kanäle (10) gleichzeitig über wenigstens teilweise denselben Zeitraum gemessen worden sind.

3. Verfahren gemäß Anspruch 1 oder 2, **dadurch gekennzeichnet, dass** der Katheter (5) nur einen einzelnen Strang (8) umfasst, an dem sich die Elektroden (7) befinden.

4. Verfahren gemäß einem der vorstehenden Ansprüche, **dadurch gekennzeichnet, dass** in der Analyseroutine (11) das Steuerungssystem (3) die Klassifizierungsroutine (17) für alle Aktivierungskandidaten (16) durchführt.

5. Verfahren gemäß einem der vorstehenden Ansprüche, **dadurch gekennzeichnet, dass** in der Klassifizierungsroutine (17) das Steuerungssystem (3) eine Timingsequenz zwischen wenigstens drei Aktivierungskandidaten (16) unterschiedlicher Kanäle (10) basierend auf den räumlichen Informationen identifiziert, und dass in der Klassifizierungsroutine (17) das Steuerungssystem (3) zwischen lokalen Aktivierungen und Fernfeldinterferenzen unterscheidet, indem eine Ausbreitungssequenz entlang der Reihenfolge der Elektroden (7) identifiziert wird und indem Aktivierungskandidaten (16) als lokale Aktivierungen klassifiziert werden, falls das Steuerungssystem (3) eine Ausbreitungssequenz identifiziert, und als Fernfeldinterferenz, falls das Steuerungssystem (3) keine Ausbreitungssequenz identifiziert.

6. Verfahren gemäß einem der vorstehenden Ansprüche, **dadurch gekennzeichnet, dass** in der Klassifizierungsroutine (17) das Steuerungssystem (3) den elektrischen Zustand basierend auf einer lokalen Aktivierungszeitdifferenz zwischen Kanälen (10) basierend auf der Reihenfolge der Elektroden (7) bestimmt.

7. Verfahren gemäß Anspruch 5 oder 6, **dadurch gekennzeichnet, dass** in der Analyseroutine (11) das Steuerungssystem (3) den elektrischen Zustand basierend auf einer Änderung einer Aktivierung oder eines Aktivierungskandidaten (16) über die zugehörige räumliche Ausbreitung bestimmt, wobei die räumliche Ausbreitung basierend auf den räumlichen Informationen und dem Timing der Aktivierung oder des Aktivierungskandidaten (16) bestimmt wird.

8. Verfahren gemäß einem der vorstehenden Ansprüche, **dadurch gekennzeichnet, dass** in einer Qualitätsschätzungsroutine das Steuerungssystem (3) einen Qualitätsindikator eines Kanals (10) basierend auf den räumlichen Informationen schätzt.

9. Verfahren gemäß Anspruch 8, **dadurch gekennzeichnet, dass** in der Qualitätsschätzungsroutine das Steuerungssystem (3) den Qualitätsindikator basierend auf einem Vergleich benachbarter Kanäle (10) schätzt, und dass das Steuerungssystem (3) in der Analyseroutine (11) nur eine Teilmenge der Kanäle (10) verwendet, ausgewählt basierend auf dem Qualitätsindikator, um den elektrischen Zustand der Zielregion (4) zu bestimmen.

10. Verfahren gemäß einem der vorstehenden Ansprüche, **dadurch gekennzeichnet, dass** in der Analyseroutine (11) das Steuerungssystem (3) eine Morphologie der lokalen Aktivierungen analysiert, um den elektrischen Isolationszustand der Zielregion (4) zu bestimmen.

11. Verfahren gemäß Anspruch 10, **dadurch gekennzeichnet, dass** in der Analyseroutine (11) das Steuerungssystem (3) die lokalen Aktivierungen in Morphologiegruppen klassifiziert und basierend auf der Verteilung lokaler Aktivierungen auf die Gruppen den elektrischen Isolationszustand bestimmt.

12. Verfahren gemäß Anspruch 11, **dadurch gekennzeichnet, dass** in der Analyseroutine (11) das Steuerungssystem (3) die lokalen Aktivierungen basierend auf einer Anzahl von charakteristischen Spitzen der lokalen Aktivierung in die Morphologiegruppen klassifiziert.

13. Verfahren gemäß Anspruch 11 oder 12, **dadurch gekennzeichnet, dass** die Morphologiegruppen eine Gruppe für lokale Aktivierungen mit einer einzelnen charakteristischen Spitze und genau zwei charakteristischen Spitzen und wenigstens zwei charakteristischen Spitzen, die durch eine vorbestimmte Zeit getrennt sind, umfasst.

14. Steuerungssystem, wobei das Steuerungssystem (3) dazu ausgelegt ist, ein intrakardiales Elektrogramm (1) zu empfangen und/oder zu messen, wobei das Steuerungssystem (3) eine lokale Einheit mit einem Prozessor und einer Benutzerschnittstelle ist, wobei das Steuerungssystem (3) einen Speicher (13) aufweist und eine Schnittstelle für einen Katheter (5) umfasst, umfassend mehrere Elektroden (7) mit einer festen Platzierungsreihenfolge der Elektroden an dem Katheter (5), wobei das Steuerungssystem (3) dazu ausgelegt ist, das Verfahren gemäß einem der vorstehenden Ansprüche durchzuführen, wobei die Routinen des Verfahrens in dem Steuerungssystem (3) vollständig als Software implementiert sind,
wobei die Reihenfolge der Elektroden (7) an dem Katheter (5) in dem Speicher (13) des Steuerungssystems (3) gespeichert ist.

## Revendications

1. Procédé d'analyse d'un électrocardiogramme (1), en particulier pour détecter un état d'isolement de la veine pulmonaire (2), par le biais d'un système de commande (3), dans lequel l'électrocardiogramme (1) a été enregistré au niveau d'une région cible (4), en particulier prédéfinie, par l'intermédiaire d'un cathéter (5) introduit dans un corps humain, dans lequel le cathéter (5) comprend de multiples électrodes (7), dans lequel de multiples canaux (10) de l'électrocardiogramme (1) ont été enregistrés par, de préférence entre, les électrodes (7), dans lequel les électrodes (7) ont un ordre de placement fixe sur le cathéter (5),
dans lequel, dans une routine d'analyse (11), le système de commande (3) détermine un état électrique de la zone cible (4) en analysant au moins trois des canaux (10), dans lequel l'ordre des électrodes (7) sur le cathéter (5) est fourni au système de commande (3) ou enregistré dans une mémoire (13) du système de commande (3) et que, dans la routine d'analyse (11), le système de commande (3) détermine l'état électrique de la région cible (4) sur la base d'informations spatiales relatives comprenant l'ordre de placement des électrodes (7) sur le cathéter (5),
dans lequel, dans la routine d'analyse (11), le système de commande (3) identifie des activations candidates (16) dans au moins trois des canaux (10), dans lequel, dans la routine d'analyse (11), le système de commande (3) effectue une routine de classification (17) pour au moins certaines des activations candidates (16) classant les activations candidates (16) en groupes, les groupes comprenant au moins un groupe affecté à des activations locales et/ou au moins un groupe affecté à une interférence en champ lointain,
**caractérisé en ce que**
dans la routine de classification (17), le système de commande (3) identifie une séquence temporelle et/ou une différence temporelle entre au moins deux activations candidates (16) de différents canaux (10) sur la base des informations spatiales pour différencier les activations locales de l'interférence en champ lointain et
que l'état électrique est un état d'isolation électrique de la région cible (4) par rapport à l'oreillette gauche et/ou droite (14) et/ou au reste de l'oreillette gauche et/ou droite (14).

2. Procédé selon la revendication 1, **caractérisé en ce que** les canaux (10) ont été mesurés en même temps sur au moins partiellement la même période.

3. Procédé selon la revendication 1 ou 2, **caractérisé en ce que** le cathéter (5) ne comprend qu'un seul brin (8) sur lequel se trouvent les électrodes (7).

4. Procédé selon l'une des revendications précédentes, **caractérisé en ce que**, dans la routine d'analyse (11), le système de commande (3) effectue la routine de classification (17) pour toutes les activations candidates (16).

5. Procédé selon l'une des revendications précédentes, **caractérisé en ce que**, dans la routine de classification (17), le système de commande (3) identifie une séquence temporelle entre au moins trois activations candidates (16) de différents canaux (10) sur la base des informations spatiales, et que, dans la routine de classification (17), le système de commande (3) différencie les activations locales de l'interférence en champ lointain en identifiant une séquence de propagation le long de l'ordre des électrodes (7) et en classant les activations candidates (16) en tant qu'activations locales si le système de commande (3) identifie une séquence de propagation et en tant qu'interférence en champ lointain si le système de commande (3) n'identifie pas de séquence de propagation.

6. Procédé selon l'une des revendications précédentes, **caractérisé en ce que**, dans la routine de classification (17), le système de commande (3) détermine l'état électrique sur la base d'une différence de temps d'activation locale entre des canaux (10) sur la base de l'ordre des électrodes (7).

7. Procédé selon la revendication 5 ou 6, **caractérisé en ce que**, dans la routine d'analyse (11), le système de commande (3) détermine l'état électrique sur la base d'un changement d'une activation ou d'une activation candidate (16) sur sa propagation spatiale, moyennant quoi la propagation spatiale est déterminée sur la base des informations spatiales et du moment de l'activation ou de l'activation candidate (16).

8. Procédé selon l'une des revendications précédentes, **caractérisé en ce que**, dans une routine d'estimation de la qualité, le système de commande (3) estime un indicateur de qualité d'un canal (10) sur la base des informations spatiales.

9. Procédé selon la revendication 8, **caractérisé en ce que**, dans la routine d'estimation de la qualité, le système de commande (3) estime l'indicateur de qualité sur la base d'une comparaison de canaux voisins (10), et que le système de commande (3), dans la routine d'analyse (11), n'utilise qu'un sous-ensemble des canaux (10) sélectionnés sur la base de l'indicateur de qualité pour déterminer l'état électrique de la région cible (4).

10. Procédé selon l'une des revendications précédentes, **caractérisé en ce, que** dans la routine d'analyse (11), le système de commande (3) analyse une morphologie des activations locales pour déterminer l'état d'isolation électrique de la région cible (4).

11. Procédé selon la revendication 10, **caractérisé en ce que**, dans la routine d'analyse (11), le système de commande (3) classe les activations locales en groupes de morphologie et détermine, sur la base de la répartition d'activations locales dans les groupes, l'état d'isolation électrique.

12. Procédé selon la revendication 11, **caractérisé en ce que**, dans la routine d'analyse (11), le système de commande (3) classe les activations locales dans les groupes de morphologie sur la base d'un nombre de crêtes caractéristiques de l'activation locale.

13. Procédé selon la revendication 11 ou 12, **caractérisé en ce que** les groupes de morphologie comprennent un groupe pour les activations locales avec une seule crête caractéristique et exactement deux crêtes caractéristiques et au moins deux crêtes caractéristiques séparées par un temps prédéfini.

14. Système de commande, dans lequel le système de commande (3) est configuré pour recevoir et/ou mesurer un électrocardiogramme (1), dans lequel le système de commande (3) est une unité locale dotée d'un processeur et d'une interface utilisateur, dans lequel le système de commande (3) possède une mémoire (13) et comprend une interface pour un cathéter (5) comprenant de multiples électrodes (7) avec un ordre fixe de placement des électrodes sur le cathéter (5), dans lequel le système de commande (3) est configuré pour effectuer le procédé selon l'une des revendications précédentes, dans lequel les routines du procédé sont entièrement mises en œuvre dans le système de commande (3) en tant que logiciel, dans lequel l'ordre des électrodes (7) sur le cathéter (5) est enregistré dans la mémoire (13) du système de commande (3).
